(19) Europäisches Patentamt
European Patent Office
Office européen des brevets

(11) Veröffentlichungsnummer: **0 097 874**
**B1**

(12) # EUROPÄISCHE PATENTSCHRIFT

(45) Veröffentlichungstag der Patentschrift: **25.11.87**

(21) Anmeldenummer: **83105856.5**

(22) Anmeldetag: **15.06.83**

(51) Int. Cl.⁴: **C 07 C 45/73**, C 07 C 45/62, C 07 C 49/633, C 07 C 49/757, C 07 C 49/443, C 07 C 49/523

(54) **Verfahren zur Herstellung von 6-exo-Alkanoyl-1,3,3-trimethyl-bicyclo(2.2.2)octan-2-onen.**

(30) Priorität: **24.06.82 DE 3223543**

(43) Veröffentlichungstag der Anmeldung: **11.01.84 Patentblatt 84/2**

(45) Bekanntmachung des Hinweises auf die Patenterteilung: **25.11.87 Patentblatt 87/48**

(84) Benannte Vertragsstaaten: **CH DE FR GB LI NL**

(56) Entgegenhaltungen:
**EP-A-0 031 965**
**FR-A-2 459 218**
**US-A-3 991 123**

**Methoden der organischen Chemie, Bd. Vc, Seite 1020, Houben-Weyl**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Pape, Frank- Friedrich, Dr., Berner Weg 34, D-6700 Ludwigshafen (DE)**
Erfinder: **Hoffmann, Werner, Dr., Ringstrasse 11c, D-6708 Neuhofen (DE)**

**EP 0 097 874 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein neues Verfahren von 6-exo-Alkanoyl-1,3,3-trimethyl-bicyclo[2.2.2]octan-2-on durch Umsetzen von 2,6,6-Trimethyl-cyclohexa-2,4-dien-1-on (II) mit Ethinylalkylketonen und katalytische Hydrierung des erhaltenen Diels-Alder-Adduktes.

Die Verfahrensprodukte sind wichtige Zwischenprodukte für die Synthese des als Duftstoffkomponente sehr begehrten Patchouli-Alkohols und seiner Derivate der Formel

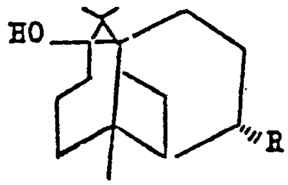

Es hat daher nicht an Versuchen gefehlt, eine besonders vorteilhafte Synthese für diese Ketone zu finden.

So sind aus der europäischen Patentanmeldung 00 31 965 sowie aus Chem. Commun. 1968, S. 1287 Verfahren zur Herstellung der bicyclischen Ketone der Formel I bekannt, bei welchen das 2,6,6-Trimethyl-cyclohexa-2,4-dien-1-on der Formel II mit Alkylvinylketonen zu den endo-konfigurierten Bicyclen der allgemeinen Formel V

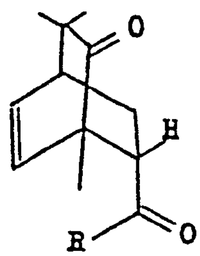

V

umgesetzt wird, welche durch Hydrieren und Equilibrieren in die gewünschten Verbindungen der Formel I überführt werden können. Da bei dieser Equilibrierung lediglich exo/endo Gemische anfallen, muß bei den in der Literatur beschriebenen Verfahren auf der letzten Stufe unvorteilhafterweise eine fraktionierte Kristallisation oder eine chromatographische Epimerentrennung vorgenommen werden. Diese Operationen dürften die Wirtschaftlichkeit des beschriebenen Verfahrens beträchtlich einschränken.

Es war däher die Aufgabe der Erfindung, ein Verfahren zur Herstellung der bicyclischen Ketone der allgemeinen Formel I zu finden, bei dem die letzte nachteilige Reaktionsstufe nicht nötig ist.

Es wurde nun überraschenderweise gefunden, daß bei der Umsetzung von II mit Ethinylalkylketonen ein bicyclisches Dien der Formel IV gebildet wird, bei dessen katalytischer Hydrierung durch ausschließlichen α-Angriff des Wasserstoffs das für die Patchouli-Alkohol-Synthese erforderliche exo-Isomere isomerenrein und in besserer Ausbeute als bei den aus der Literatur bekannten Verfahren erhalten wird.

Gegenstand der Erfindung ist dementsprechend ein Verfahren zur Herstellung von 6-exo-Alkanoyl-1,3,3-trimethyl-bicyclo[2.2.2]octan-2-onen der allgemeinen Formel I

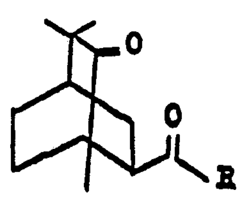

(I)

in der R für Wasserstoff oder einen gesättigten, verzweigten oder unverzweigten, aliphatischen Kohlenwasserstoffrest mit 1 bis 6 C-Atomen steht, das dadurch gekennzeichnet ist, daß man

A. 2,6,6-Trimethyl-cyclohexa-2,4-dien-1-on der Formel II

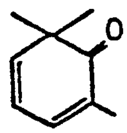

(II)

mit einem Ethinylketon der allgemeinen Formel III

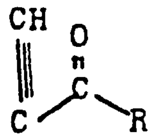

(III),

in der R die oben angegebene Bedeutung hat, gegebenenfalls in einem inerten Lösungsmittel auf Temperaturen von 100 bis 200, vorzugsweise 100 bis 150° C erhitzt und

B. das erhaltene ungesättigte bicyclische Keton der allgemeinen Formel IV

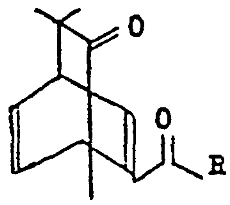

(IV)

in Gegenwart von Edelmetallkatalysatoren katalytisch hydriert.

Das erfindungsgemäße Verfahren ist somit um die letzte und kritischste Reaktionsstufe kürzer.

Die für das erfindungsgemäße Verfahren benötigten Ethinylalkylketone können durch Ethinylierung aliphatischer Aldehyde und Oxidation der resultierenden Alkohole auf einfache Weise hergestellt werden. Gegenüber den bei dem bekannten Verfahren als Ausgangsverbindungen verwendeten Vinylalkylketonen zeichnen sie sich durch eine wesentlich geringere Polymerisationsempfindlichkeit aus.

II wird vorteilhaft in Form seines Dimeren eingesetzt, da dieses die einzige lagerungsstabile Form ist und bei höheren Temperaturen sowieso in die Monomeren aufspaltet.

Zur Umsetzung von II mit den Ketonen der Formel III zu den Bicyclooctadiendionen der Formel IV werden die Komponenten einfach miteinander oder aber in einem inerten höhersiedenden Lösungsmittel erhitzt. Hierbei werden pro Mol II mindestens äquimolare Mengen, vorzugsweise 1 bis 1,2 Mol des Ketons III zugesetzt.

Ein Lösungsmittel ist vor allem nicht nötig, wenn die Reaktionskomponenten im Autoklaven bei autogenem Druck auf die Reaktionstemperatur erhitzt werden. Man kann aber auch in einem für Diels-Alder-Synthesen üblichen inerten Lösungsmittel, wie Benzol, Toluol, Xylol oder 1,4-Dioxan arbeiten. Das Lösungsmittel verwendet man dann in etwa der gleichen bis etwa der doppelten Gewichtsmenge.

Von den bei dieser Diels-Alder-Reaktion möglichen Konstitutionsisomeren IV und seinem 5-Isomeren VI

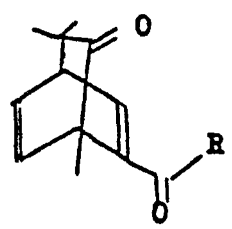

(IV)

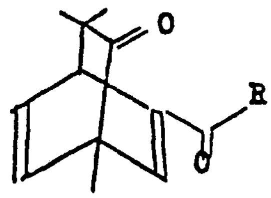

(VI)

wurden bei Verbindungen, in denen R eine Methylgruppe bedeutet, die 5-Isomeren nicht isoliert. Mit steigendem induktiven Effekt (bzw. steigender sterischer Hinderung) des Substituenten R nimmt der Anteil an dem unerwünschten 5-Isomeren zu. Das Verhältnis der Konstitutionsisomeren ist außerdem in gewissem Maße von der Wahl des Lösungsmittels abhängig. Dieser Effekt, der in diesem Fall zugunsten des gewünschten Verfahrensproduktes genutzt werden kann, ist überraschend, da pericyclische Reaktionen normalerweise nicht lösungsmittelabhängig sind.

So wird z. B. bei Verwendung des etwas polareren 1,4-Dioxans anstelle der apolaren aprotonischen Kohlenwasserstoffe das Isomerenverhältnis deutlich zur Seite der gewünschten Konstitutionsisomeren verschoben.

Für R = Hexyl beträgt beispielsweise das Verhältnis von IV:VI beim Arbeiten in Toluol = 87,5 : 12,5, in 1,4-Dioxan dagegen 94,3 : 5,7. Für Verbindungen mit R = Isopropyl sind die Verhältnisse analog. Sie betragen

| | | | | |
|---|---|---|---|---|
| beim Arbeiten | in Xylol: | IV:VI = 81,8 | : | 18,2 |
| | in Dioxan: | = 88 | : | 12 |
| | ohne Lösungsmittel: | = 88 | : | 12 |
| | und in Methanol: | 91 | : | 9. |

Der Verlauf der Umsetzung kann mit den üblichen analytischen Methoden verfolgt werden. Die zur quantitativen Umsetzung von II benötigte Reaktionszeit beträgt im allgemeinen etwa 3 Stunden.

Die Aufarbeitung des Reaktionsansatzes erfolgt durch Abdestillieren des Lösungsmittels. Der verbleibende Rückstand kann entweder durch Destillation gereinigt werden, oder aber - im Falle kristalliner Reaktionsprodukte - durch Lösen in heißem Petrolether, Filtrieren und anschliessende Kristallisation. Aus der Lösung kristallisieren die bicyclischen Octadienone IV in reiner Form aus.

Die katalytische Hydrierung der Bicyclooctadiendione IV erfolgt vorteilhaft in polar-protischen Lösungsmitteln wie Methanol, Ethanol oder Isopropanol, insbesondere in Methanol. Bei Anwendung von Normaldruck bis Drucken von etwa 30 bar und Temperaturen von etwa 100°C reagieren die Bicyclooctadiendione IV bei Verwendung von Edelmetallkatalysatoren, wie Pd- oder Pt-Katalysatoren glatt zu den Bicyclooctandionen der Formel I. In Essigsäureethylester als Lösungsmittel gelingt dagegen interessanterweise eine selektive Hydrierung der Doppelbindung in 7-Stellung.

Mit Hilfe des erfindungsgemäßen Verfahrens erhält man die als wichtige Zwischenprodukte für die Synthese des als Duftstoffkomponente sehr begehrten Patchouli-Alkohols und seiner Derivate interessanten bicyclischen Ketone der allgemeinen Formel I in einfacher Weise und mit besseren Ausbeuten als nach den bekannten Verfahren.

**Beispiel 1**

6-Acetyl-1,3,3-trimethyl-bicyclo[2.2.2]octan-2-on

A. Unter Rühren wurden zu der siedenden Lösung von 95,2 g (0,7 mol) dimerem 2,6,6-Trimethyl-cyclohexa-2,4-dien-1-on (II) in 300 ml Xylol 53 g (0,77 mol) Butinon getropft. Nach beendeter Zugabe wurde 3 h unter Rühren und unter Rückflußkühlung zum Sieden erhitzt.

Anschließend wurde das Xylol abdestilliert, das Reaktionsprodukt in 0,5 l Petrolether aufgenommen und zum Sieden erhitzt. Die heiße Petroletherlösung wurde filtriert. Aus der erkalteten Lösung kristallisierten 97 g (0,48 mol) 6-Acetyl-1,3,3-trimethyl-bicyclo[2.2.2]-octan-5,7-dien-2-on (IVa) in Form farbloser Prismen vom Festpunkt F. 70 bis 72°C aus. Das entspricht einer Ausbeute von 69 % der Theorie.

Ba. 28 g des erhaltenen 6-Acetyl-1,3,3-trimethyl-bicyclo-[2.2.2]octa-5,7-dien-2-ons wurden in 150 ml Isopropanol gelöst, die Lösung mit 1 g Pd/C (5 %-ig) versetzt und bei 100°C und 30 bar 5 h im Autoklaven hydriert. Anschließend wurde der Katalysator abfiltriert und das Reaktionsprodukt vom Lösungsmittel befreit. Es verblieben 27 g (95 %) 6-exo-Acetyl-1,3,3-trimethyl-bicyclo[2.2.2]octan-2-on (Ia) als farbloses, langsam kristallisierendes Öl vom Schmelzpunkt F = 45 bis 48°C.

Bb. 5 g IVa wurden mit 0,1 g Pt/C (5 %ig) unter sonst gleichen Bedingungen wie in Ba) dydriert. Man erhielt 5 g Ia.

Bc. 5 g IVa wurden in Methanol gelöst, mit 0,1 g Pd/C (5 %-ig) versetzt und im Autoklaven bei 100°C und 30 bar hydriert. Man erhielt 5 g Ia.

Bd. 5 g IVa wurden in Methanol gelöst und mit 0,1 g Pt/C (5 %-ig) versetzt. Nach Hydrieren bei 100°C und 30 bar verblieben 5 g Ia.

**Beispiel 2**

6-exo-Isobutyryl-1,3,3-trimethyl-bicyclo[2.2.2]octan-2-on

Aa. Zu der siedenden Lösung von 27,2 g (0,2 mol) des dimeren 2,6,6-Trimethyl-cyclohexa-2,4-dien-1-on (II) in 100 ml Xylol wurden unter Rühren 21,2 g (0,22 mol) Ethinylisopropylketon getropft. Nach beendeter Zugabe wurde 3 h unter Rühren und unter Rückflußkühlung zum Sieden erhitzt. Anschließend wurde das Xylol abdestilliert. Durch Kugelrohrdestillation ($K_p$ = 81 bis 100°C/0,005 mbar) des Rückstandes erhielt man 41,5 g eines Gemisches aus 6-Isobutyryl-1,3,3-trimethyl-bicyclo[2.2.2]octa-5,7-dien-2-ons (IVb) und seinem 5-Isobutyryl-isomeren im Verhältnis 9:2.

Die Isomeren können durch Kristallisation aus Petrolether getrennt werden. Das gewünschte 6-Isobutyryl-derivat fällt hierbei in Form von weißen Kristallen vom Schmelzpunkt F = 60 bis 63° C an.

Ab. Wurde als Lösungsmittel anstelle von Xylol Dioxan verwendet und die Reaktionszeit auf 5 h verlängert, so erhielt man unter sonst gleichen Reaktionsbedingungen in praktisch quantitativer Ausbeute ein Gemisch aus 88 % IVb und 12 % des konstitutionsisomeren 5-Isobutyrylderivats.

Ac. Wurde als Lösungsmittel anstelle von Xylol Methanol verwendet und das Reaktionsgemisch im Autoklaven 3 h auf 140° C erhitzt, so erhielt man bei üblicher Aufarbeitung in etwa 83 %-iger Ausbeute ein Gemisch aus 91 % IVb und 9 % des konstitutionsisomeren 5-Isobutyrylderivats.

Ad. Eine Mischung aus 27,2 g II und 21,1 g Ethinylisopropylketon wurde 3 h im Autoklaven auf 130° C erhitzt. Man erhielt in praktisch quantitativer Ausbeute ein Gemisch aus IVb und seinem 5-Isobutyrylisomeren im Verhältnis 88:12.

B. 10 g IVb wurden in 100 ml Methanol gelöst, mit 0,1 g Pd/C (5 %-ig) versetzt und 3 h bei 30 bar und 100° C hydriert. Durch Filtrieren, Abdestillieren des Methanols und Destillieren im Kugelrohr erhielt man 7,2 g 6-exo-Isobutyryl-1,3,3-trimethyl-bicyclo[2.2.2]octan-2-on als farbloses Öl vom Siedepunkt Kp = 120° C/0,01 mbar.

**Beispiel 3**

6-exo-(2'-Metdyl-butyryl)-1,3,3-trimethyl-bicyclo[2.2.2]-octan-2-on

A. Analog Beispiel 2 Aa wurden 27,2 g dimeres II mit 24,2 g 4-Methyl-hex-1-in-3-on in Xylol umgesetzt. Destillation des Reaktionsproduktes ergab ein Gemisch vom Siedepunkt Kp = 103 bis 108° C/0,01 mbar, das zu 4 Teilen aus 6-exo-(2'-Methyl-butyryl)-1,3,3-trimethyl-bicyclo[2.2.2]octa-5,7-dien-1-on (IVc) und 1 Teil aus dem entsprechenden 5-Isomeren bestand. Die Produkte sind Diastereomerengemische bezüglich des Chiralitätszentrums in der Seitenkette.

B. 10 g des gemäß 3A erhaltenen Gemisches wurden analog Beispiel 2B hydriert. Durch Kugelrohrdestillation des Reaktionsproduktes erhielt man 7,8 g eines im wesentlichen 6-exo-(2'-Methyl-butyryl)-1,3,3-trimethyl-bicyclo[2.2.2]octan-2-on enthaltendes Gemisch als gelbes Öl vom Siedepunkt Kp = 150 bis 160° C/0,3 mbar.

**Beispiel 4**

6-exo-Propionyl-1,3,3-trimethyl-bicyclo[2.2.2]octan-2-on

A. Analog Beispiel 1A wurden 37,3 g dimeres II mit 25 g Ethinyl-ethylketon in 200 ml Xylol umgesetzt. Destillation des erhaltenen Reaktionsproduktes ergab 49 g eines Gemisches aus 6-Propionyl-1,3,3-trimethyl-bicyclo[2.2.2]octa-5,7-dien-2-on (IVd) und seinem 5-Propionyl-isomeren im Verhältnis 7:1.

B. 10 g des gemäß A erhaltenen Produktes wurden analog Beispiel 2B hydriert. Nach Kugelrohrdestillation verblieben 7,8 g eines im wesentlichen 6-exo-Propionyl-1,3,3-trimethyl-bicyclo[2.2.2]octan-2-on enthaltendes Gemisch als farbloses Öl vom Siedepunkt Kp = 120 bis 125° C/0,005 mbar.

**Beispiel 5**

6-exo-Heptanoyl-1,3,3-trimethyl-bicyclo[2.2.2]octan-2-on

Aa. Analog Beispiel 1A wurden 27,2 g des dimeren II mit 30,4 g Ethinyl-hexylketon in 200 ml Xylol umgesetzt. Durch Kugelrohrdestillation des Reaktionsproduktes erhielt man 47 g eines 7:1 Gemisches aus 6-Heptanoyl-1,3,3-trimethyl-bicyclo[2.2.2]octa-5,7-dien-2-on (IVe) und seinem 5-Heptanoylisomeren.

Ab. Wurde anstelle von Xylol 200 ml 1,4-Dioxan als Lösungsmittel verwendet, so erhielt man unter sonst gleichen Reaktionsbedingungen ein Gemisch bestehend aus 94,3 % IVe und 5,7 % seines 5-Heptanoyl-Isomeren. Die Ausbeute betrug 76 % der Theorie.

B. 10 g des gemäß Beispiel 5Ab erhaltenen Gemisches wurden analog Beispiel 2B hydriert. Man erhielt 8,7 g

eines im wesentlichen 6-exo-Heptanoyl-1,3,3-trimethyl-bicyclo[2.2.2]octan-2-on enthaltenden Gemisches als gelbes Öl vom Siedebereich Kp = 120 bis 150° C/0,05 mbar.

**Patentanspruch**

Verfahren zur Herstellung von 6-exo-Alkanoyl-1,3,3-trimethyl-bicyclo[2.2.2]octan-2-onen der allgemeinen Formel I

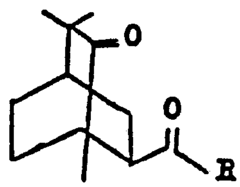

(I)

in der R für Wasserstoff oder einen gesättigten, verzweigten oder unverzweigten aliphatischen Kohlenwasserstoffrest mit 1 bis 6 C-Atomen steht, <u>dadurch gekennzeichnet,</u> daß man

A. 2,6,6-Trimethyl-cyclohexa-2,4-dien-1-on der Formel II

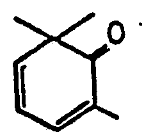

(II)

mit einem Ethinylketon der allgemeinen Formel III

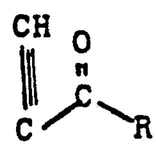

(III),

in der R die oben angegebene Bedeutung hat, gegebenenfalls in einem inerten Lösungsmittel auf Temperaturen von 100 bis 200° C erhitzt und

B. das erhaltene ungesättigte bicyclische Keton der allgemeinen Formel IV

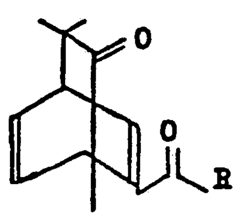

(IV)

in Gegenwart von Edelmetallkatalysatoren katalytisch hydriert.

**Claim**

A process for the preparation of a 6-exo-alkanoyl-1,3,3-trimethyl-bicyclo[2,2,2]octan-2-one of the general formula I

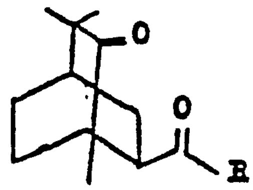

(I),

where R is hydrogen or a saturated, branched or linear aliphatic hydrocarbon radical of 1 to 6 carbon atoms, wherein

A. 2,6,6-trimethyl-cyclohexa-2,4-dien-1-one of the formula II

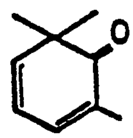

(II)

is heated together with an ethynyl ketone of the general formula III

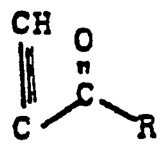

(III),

where R has the above meanings, in the presence or absence of an inert solvent, to a temperature of from 100 to 200° C, and

B. the resulting unsaturated bicyclic ketone of the general formula IV

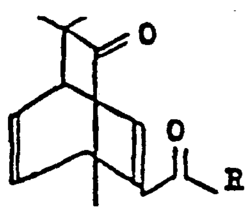

(IV)

is catalytically hydrogenated in the presence of a noble metal catalyst.

**Revendication**

Procéde de préparation de 6-exo-alcanoyl-1,3 3-triméthyl-bicyclo[2.2.2]octane-2-ones de la formule générale I

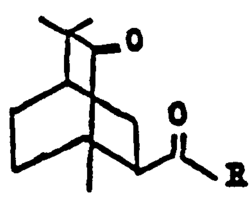

(I)

dans laquelle R est mis pour un atome d'hydrogène ou pour un radical hydrocarboné aliphatique, saturé, ramifié ou non ramifié, contenant 1 à 6 atomes de carbone, caractérisé en ce que

A. on chauffe la 2,6,6-triméthyl-cyclohexa-2,4-diène-1-one de la formule II

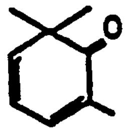

(II)

avec une éthinylcétone de la formule générale III

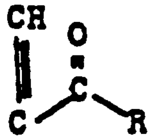

(III),

dans laquelle R a les significations susindiquées, éventuellement dans un solvant inerte, à des temperatures de 100 à 200°C, et

B. on hydrogène catalytiquement, en présence de catalyseurs contenant des métaux nobles, la cétone bicyclique insaturée obtenue de la formule générale IV

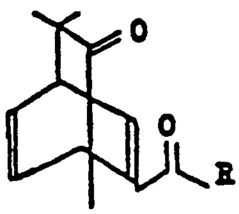

(IV)